# EUROPEAN PATENT APPLICATION

(11) **EP 3 533 865 A1**
(43) Date of publication of application: **04.09.2019**
(21) Application number: 17866130.2
(22) Date of filing: 27.10.2017
(51) Int. Cl.: C12N 5/071, A61P 1/16, C12N 1/00, C12N 7/00, C12Q 1/02, A61K 35/407

(54) **METHOD FOR PREPARING HUMAN HEPATOCYTE PROGENITOR CELLS**

(30) Priority: 28.10.2016 JP 2016212285
(71) Applicant: National Cancer Center, Tokyo 104-0045 (JP); Interstem Co., Ltd., Tokyo 105-0022 (JP)
(72) Inventor: KATSUDA,Takeshi, Tokyo 104-0045 (JP); OCHIYA,Takahiro, Tokyo 104-0045 (JP); YAMADA,Tetsumasa, Tokyo 105-0022 (JP)
(74) Representative: Cabinet Beau de Loménie
(86) International application number: PCT/JP2017/038907
(87) International publication number: WO 2018/079714

(57) **Abstract**

Provided is a method for reprogramming human mature hepatocytes in vitro into liver progenitor cells capable of self-renewal. Disclosed is a method for preparing human liver progenitor cells, comprising culturing human mature hepatocytes in a medium containing serum, A-83-01, and CHIR99021.

## Description

### TECHNICAL FIELD

The present invention relates to a method for preparing human liver progenitor cells.

### BACKGROUND ART

Liver transplantation is the only currently effective treatment for end-stage liver diseases, but has a problem that there is an absolute shortage of liver donors. To replace this problem, attempts have been continued to induce differentiation of hepatocytes from iPS cells such that differentiated hepatocytes are used for transplantation treatment.

However, since iPS cells have totipotency and high proliferative capacity, there are reports saying that their transplantation into immunocompromised mice leads to the formation of teratomas. Thus, when hepatocytes that have been generated from iPS cells are contaminated with undifferentiated iPS cells even in small amounts and used for transplantation, there is a risk that tumors may be developed after their transplantation. In addition, there have not yet been established efficient methods in which iPS cells are used to induce differentiation into endodermal cells, such as, liver cells, and it is currently not possible to obtain hepatocytes from iPS cells in amounts as much as liver function can be replaced by the resultant hepatocytes.

On the other hand, liver progenitor cells that are present in the adult liver in a very small amount have been considered to be used as a source of hepatocytes. Recent studies have also revealed the fact that mature hepatocytes are reprogrammed, during chronic hepatitis, into bipotent progenitor cells that are capable of differentiation into hepatocytes and biliary epithelial cells (Non-Patent Documents 1 to 4).

### PRIOR ART DOCUMENTS

### NON-PATENT DOCUMENTS

Non-Patent Document 1: Yanger K., et. al., Genes & Development, pp. 719-724, 27, 2013
Non-Patent Document 2: Tanimizu N., et. al., Journal of Biological Chemistry, pp. 7589-7598, 289 (11), 2014
Non-Patent Document 3: Yimlamai D., et. al., Cell, pp. 1324-1338, 157, 2014
Non-Patent Document 4: Tarlow B. D., et. al., Cell Stem Cell, pp. 605-618, 15, 2014

### SUMMARY OF THE INVENTION

### PROBLEMS TO BE SOLVED BY THE INVENTION

It is an object of the present invention to provide a method for reprogramming human mature hepatocytes in vitro into human liver progenitor cells capable of self-renewal.

### MEANS FOR SOLVING THE PROBLEMS

The present inventors have made extensive investigations to solve the problem, with the result that it was found that human mature hepatocytes can be reprogrammed into human liver progenitor cells capable of self-renewal by culturing them in vitro in a medium containing serum, A-83-01 (a TGF-β signaling inhibitor), and CHIR99021 (a GSK3 inhibitor), thereby completing the present invention.

Accordingly, the present invention provides a method for preparing human liver progenitor cells, as described below.
Item 1. A method for preparing human liver progenitor cells, comprising culturing human mature hepatocytes in a medium containing serum, A-83-01, and CHIR99021.
Item 2. The method for preparing human liver progenitor cells according to item 1, wherein the mature hepatocytes are derived from an infant or a toddler.
Item 3. The method for preparing human liver progenitor cells according to item 1 or 2, wherein the serum is fetal bovine serum.
Item 4. A human liver progenitor cell prepared by culturing human mature hepatocytes in a medium containing serum, A-83-01, and CHIR99021.
Item 5. A mature hepatocyte derived from the human liver progenitor cell according to item 4.
Item 6. A method for screening test substances, comprising using the mature hepatocyte according to item 5.
Item 7. A method for culturing a hepatitis virus, comprising using the mature hepatocyte according to item 5.
Item 8. A model animal of human liver in which the mature hepatocyte according to item 5 has been transplanted into a non-human mammal.
Item 9. A kit for evaluating metabolism and/or hepatotoxicity of a test substance using human liver progenitor cells or mature hepatocytes, the kit comprising human liver progenitor cells prepared by culturing human mature hepatocytes in a medium containing serum, A-83-01, and CHIR99021, or mature hepatocytes derived from the human liver progenitor cells.

### EFFECT OF THE INVENTION

According to the present invention, a method can be provided for reprogramming human mature hepatocytes in vitro into liver progenitor cells capable of self-renewal.

### BRIEF DESCRIPTION OF THE DRAWINGS

Fig. 1 represents phase-contrast micrographs showing results when human mature hepatocytes were cultured in AC-F medium (A), YAC-F medium (B), or YAC medium (C).
Fig. 2 represents phase-contrast micrographs showing results when human mature hepatocytes were cultured in AC-F medium.
Fig. 3 represents phase-contrast micrograph showing results when human mature hepatocytes were cultured in AC-F medium or FBS medium.
Fig. 4 represents phase-contrast micrograph showing results when human mature hepatocytes were cultured in AC-F medium or FBS medium.
Fig. 5 represents a graph showing changes over time of human-specific albumin in blood in cDNA-uPA/SCID mice after administration of liver progenitor cells.
Fig. 6 represents an immunostaining photograph showing the expression of human CYP2C9 in the medial right lobe of the liver of a cDNA-uPA/SCID mouse 70 days after administration of liver progenitor cells.
Fig. 7 represents an immunostaining photograph showing the expression of human CYP2C9 in the medial left lobe of the liver of a cDNA-uPA/SCID mouse 70 days after administration of liver progenitor cells.
Fig. 8 represents a graph showing changes over time of human-specific albumin in blood in TK-NOG mice after administration of liver progenitor cells.
Fig. 9 represents an immunostaining photograph showing the expression of human CYP2C9 in the liver of a TK-NOG mouse 60 days after administration of liver progenitor cells.
Fig. 10 represents an immunostaining photograph showing the expression of human CYP2C9 in the liver of a TK-NOG mouse 60 days after administration of liver progenitor cells.
Fig. 11 represents a graph showing the activity of the metabolic enzyme CYP1A2 in liver progenitor cells of the present invention or mature hepatocytes differentiated therefrom (differentiated mature hepatocytes).
Fig. 12 represents a graph showing the ratio of the activity of the metabolic enzyme CYP1A2 with and without Omeprazole induction, in liver progenitor cells of the present invention or mature hepatocytes differentiated therefrom (differentiated mature hepatocytes).
Fig. 13 represents a graph showing the activity of the metabolic enzyme CYP3A4 in liver progenitor cells of the present invention or mature hepatocytes differentiated therefrom (differentiated mature hepatocytes).
Fig. 14 represents a graph showing the ratio of the activity of the metabolic enzyme CYP3A4 with and without Phenobarbital induction, in liver progenitor cells of the present invention or mature hepatocytes differentiated therefrom (differentiated mature hepatocytes).
Fig. 15 represents a graph showing the amount of expression of the ALB gene in liver progenitor cells of the present invention or mature hepatocytes differentiated therefrom (differentiated mature hepatocytes).
Fig. 16 represents a graph showing the amount of expression of the TAT gene in liver progenitor cells of the present invention or mature hepatocytes differentiated therefrom (differentiated mature hepatocytes).
Fig. 17 represents a graph showing the amount of expression of the TDO2 gene in liver progenitor cells of the present invention or mature hepatocytes differentiated therefrom (differentiated mature hepatocytes).
Fig. 18 represents a graph showing the amount of expression of the TTR gene in liver progenitor cells of the present invention or mature hepatocytes differentiated therefrom (differentiated mature hepatocytes).
Fig. 19 represents a graph showing the amount of expression of the G6PC gene in liver progenitor cells of the present invention or mature hepatocytes differentiated therefrom (differentiated mature hepatocytes).
Fig. 20 represents a graph showing the amount of expression of the NTCP gene in liver progenitor cells of the present invention or mature hepatocytes differentiated therefrom (differentiated mature hepatocytes).
Fig. 21 represents a graph showing the amount of expression of the CYP1A2 gene in liver progenitor cells of the present invention or mature hepatocytes differentiated therefrom (differentiated mature hepatocytes).
Fig. 22 represents a graph showing the amount of expression of the CYP2B6 gene in liver progenitor cells of the present invention or mature hepatocytes differentiated therefrom (differentiated mature hepatocytes).
Fig. 23 represents a graph showing the amount of expression of the CYP2C9 gene in liver progenitor cells of the present invention or mature hepatocytes differentiated therefrom (differentiated mature hepatocytes).
Fig. 24 represents a graph showing the amount of expression of the CYP2C19 gene in liver progenitor cells of the present invention or mature hepatocytes differentiated therefrom (differentiated mature hepatocytes).
Fig. 25 represents a graph showing the amount of expression of the CYP2D6 gene in liver progenitor cells of the present invention or mature hepatocytes differentiated therefrom (differentiated mature hepatocytes).
Fig. 26 represents a graph showing the amount of expression of the CYP3A4 gene in liver progenitor cells of the present invention or mature hepatocytes differentiated therefrom (differentiated mature hepatocytes).
Fig. 27 represents a graph showing the amount of expression of the CYP7A1 gene in liver progenitor cells of the present invention or mature hepatocytes differentiated therefrom (differentiated mature hepatocytes).
Fig. 28 represents a photograph of liver progenitor cells according to an embodiment of the present invention prior to transplantation into a cDNA-uPA/SCID mouse.
Fig. 29 represents a photograph of cells that were removed from a cDNA-uPA/SCID mouse having liver progenitor cells according to an embodiment of the present invention transplanted thereinto and cultured for four days.
Fig. 30 represents a graph showing the activity of CYP1A2 in hepatocytes removed from the cDNA-uPA/SCID mice.
Fig. 31 represents a graph showing the activity of CYP3A4 in hepatocytes removed from the cDNA-uPA/SCID mice.
Fig. 32 represents a photograph of liver progenitor cells according to another embodiment of the present invention prior to transplantation into a cDNA-uPA/SCID mouse.
Fig. 33 represents a photograph of cells that were removed from a cDNA-uPA/SCID mouse having liver progenitor cells according to another embodiment of the present invention transplanted thereinto and cultured for four days.
Fig. 34 represents a graph showing the activity of CYP1A2 in hepatocytes removed from the cDNA-uPA/SCID mice.
Fig. 35 represents a graph showing the activity of CYP3A4 in hepatocytes removed from the cDNA-uPA/SCID mice.
Fig. 36 represents a photograph of liver progenitor cells according to another embodiment of the present invention prior to transplantation into a cDNA-uPA/SCID mouse.
Fig. 37 represents a photograph of cells that were removed from a cDNA-uPA/SCID mouse having liver progenitor cells according to another embodiment of the present invention transplanted thereinto and cultured for four days.
Fig. 38 represents a graph showing the activity of CYP1A2 in hepatocytes removed from the cDNA-uPA/SCID mice.
Fig. 39 represents a graph showing the activity of CYP3A4 in hepatocytes removed from the cDNA-uPA/SCID mice.

### MODE FOR CARRYING OUT THE INVENTION

The present invention relates to a method for preparing human liver progenitor cells, comprising culturing human mature hepatocytes in a medium containing serum, A-83-01, and CHIR99021.

### [Human mature hepatocytes]

The human mature hepatocytes that are used in the present invention can be obtained from biogenic liver tissues according to any known method. In the specification, by biogenic liver tissue is meant a liver tissue obtained from a human liver after birth. An individual for the source of biogenic liver tissues may be alive or dead. The individual employed as a source of biogenic liver tissues is not limited to a particular age, and preferably is in their twenties or younger, further preferably is 10 years of age or younger, more preferably is an infant or an toddler (0 to 7 years of age), and most preferably is an infant (0 to 2 years of age).

The human mature hepatocytes that are used in the method of the present invention are can be in any form, as long as they have characteristics as exhibited by mature hepatocytes, and may be those that have been subjected to freeze storage after being obtained from a biogenic liver tissue, or those that have been repeatedly subjected to freeze storage and thawing as appropriate after being obtained from a biogenic liver tissue. The human mature hepatocytes may be human-derived mature hepatocytes that are commercially available.

In addition, the human mature hepatocytes include fully differentiated hepatocytes that are incapable of in vitro proliferation.

The human mature hepatocytes may be hepatocytes that have been subjected to induction of differentiation from iPS (induced pluripotent stem) cells, ES (embryonic stem) cells, and the like.

### [Serum]

The serum that is used in the present invention includes, for example, human serum, fetal bovine serum (FBS), bovine serum, calf serum, goat serum, horse serum, porcine serum, sheep serum, rabbit serum, rat serum, and others. Preference is given to FBS, calf serum, and bovine serum, with FBS being further preferable. In addition, the serum may be a material derived from serum components, such as albumin (for example, bovine, porcine, human, dog, rabbit, rat, mouse, chicken albumins), and human platelet lysate. The serum may be commercially available.

The content of serum used in the present invention is from 0.1 v/v% to 30 v/v%, preferably from 1 v/v% to 20 v/v%, further preferably from 5 v/v% to 15 v/v%, still more preferably from 8 v/v% to 12 v/v%, most preferably 10 v/v%, relative to the volume of the whole medium.

### [A-83-01]

A-83-01 (CAS No. 909910-43-6) is a TGF-β signaling inhibitor and is capable of selectively inhibiting TGF-β type I/activin receptor like kinase (ALK5), type I activin/nodal receptor kinase (ALK4), and type I nodal receptor kinase (ALK7). A-83-01 is also known as 3-(6-Methyl-2-pyridinyl)-N-phenyl-4-(4-quinolinyl)-1H-pyrazole-1-carbothioamide. A-83-01 is available from, for example, Wako Pure Chemical Industries, Ltd., without limitation thereto.

The content of A-83-01 used in the present invention is from 0.0001 µM to 5 µM, preferably from 0.001 µM to 2 µM, further preferably from 0.01 µM to 1 µM, still more preferably from 0.05 µM to 0.7 µM, most preferably 0.5 µM, relative to the volume of the whole medium.

### [CHIR99021]

CHIR99021 (CAS No. 252917-06-9) is an inhibitor of GSK-3β (Glycogen Synthase Kinase 3β) and is currently known as the most selective inhibitor of it. CHIR99021 is also known as 6-[[2-[[4-(2,4-dichlorophenyl)-5-(5-methyl-1H-imidazol-2-yl)-2-pyrimidinyl]amino]ethyl]amino]-3-pyridinecarbonitrile. CHIR99021 is available from, for example, Wako Pure Chemical Industries, Ltd., without limitation thereto.

The content of CHIR99021 used in the present invention is from 0.001 µM to 20 µM, preferably from 0.01 µM to 10 µM, further preferably from 0.1 µM to 5 µM, still more preferably from 0.3 µM to 4 µM, most preferably is 3 µM, relative to the volume of the whole medium.

### [ROCK inhibitors]

In the present invention, the medium in which human mature hepatocytes are cultured may further contain a ROCK inhibitor. By way of example, the ROCK inhibitor is, but is not limited to, Y-27632 (CAS No. 146986-50-7), GSK 269962 (CAS No. 850664-21-0), Fasudil hydrochloride (CAS No. 105628-07-7), H-1152 (CAS No. 871543-07-6), with Y-27632 being preferred. Y-27632 is a selective and potent ROCK (Rho-associated coiled forming kinase/Rho-binding kinase) inhibitor. Y-27632 is also known as trans-4-[(1R)-1-aminoethyl]-N-4-pyridinyl-cyclohexanecarboxamide. Y-27632 may be in a free form, or may be in the form of a salt such as a hydrochloride or a sulfate, or may be a hydrate. GSK269962A is also known as N-[3-[[2-(4-Amino-1,2,5-oxadiazol-3-yl)-1-ethyl-1H-imidazo[4,5-c]pyridin-6-yl]oxy]phenyl]-4-[2-(4-morpholinyl)ethoxy]benzamide. Fasudil hydrochloride is also known as 1-(5-Isoquinolinesulfonyl)homopiperazine Dihydrochloride. Fasudil hydrochloride may be in a free form, or may be in the form of a salt such as a hydrochloride or a sulfate, or may be a hydrate. H-1152 is also known as (S)-(+)-2-Methyl-1-[(4-methyl-5-isoquinolinyl)sulfonyl]-hexahydro-1H-1,4-diazepine dihydrochloride. Y-27632 is available from, for example, Wako Pure Chemical Industries, Ltd., without limitation thereto. SK269962A is available from, for example, Axon medchem, without limitation thereto. Fasudil hydrochloride is available from, for example, Tocris Bioscience, without limitation thereto. H-1152 is available from, for example, Wako Pure Chemical Industries, Ltd., without limitation thereto.

The content of a ROCK inhibitor used in the present invention is from 0.001 µM to 50 µM, preferably from 0.01 µM to 30 µM, further preferably from 0.1 µM to 20 µM, still more preferably from 1 µM to 15 µM, most preferably 10 µM, relative to the volume of the whole medium.

In the present invention, the medium in which human mature hepatocytes are cultured can be added with, for example, one or more of various genes, or a gene product(s) thereof, such as protein(s) and mRNA(s), or one or more agents, known in the technology of inducing ES cells, iPS cells, and others. Also, one or more of various genes, or a gene product(s) thereof, such as protein(s) and mRNA(s), known in the technology of inducing ES cells, iPS cells, and others can be expressed or introduced in mammalian cells.

In the present invention, the medium in which human mature hepatocytes are cultured can be added with an agent, compound, or antibody that is known to induce ES cells, iPS cells, or the like, in order to improve the efficiency of induction to liver progenitor cells. For example, the medium can be added with a small molecule inhibitor for three of FGF receptor tyrosine kinase, the MEK (mitogen-activated protein kinase)/ERK (extracellular signal-regulated kinases 1 and 2) pathway, and GSK (glycogen synthase kinase)-3 [SU5402 and PD184352]; a small molecule inhibitor for the MEK/ERK pathway and GSK3 [PD0325901]; a small molecule compound that is an inhibitor of histone methyltransferase G9a [BIX-01294 (BIX)]; azacytidine; trichostatin A (TSA); 7-hydroxyflavone; lysergic acid ethylamide; kenpaullone; an inhibitor of TGF-β receptor I kinase/activin-like kinase 5 (ALK5) [EMD 616452]; an inhibitor of TGF-β receptor 1 (TGF-βR1) kinase [E-616452 and E-616451]; an inhibitor of Src-family kinases [EI-275]; thiazovivin; PD0325901; SU5402; PD184352; SB431542; an anti-TGF-β neutralizing antibody; TNr5a2; a p53 inhibitory compound; a siRNA against p53; and/or a p53 pathway inhibitor, without limitation thereto.

Additionally, a microRNA that is used for generating ES cells, iPS cells, etc. can be further employed in the medium in which human mature hepatocytes are cultured, in order to improve the efficiency of induction to liver progenitor cells.

In the present invention, inhibitors or antibodies that inhibit or neutralize, respectively, the activity of TGF-β or the like can also be used in the medium in which human mature hepatocytes are cultured. Inhibitors of TGF-β include, for example, TGF-βRI inhibitors, and TGF-βRI kinase inhibitors.

When cultured according to the present invention, human mature hepatocytes are also preferably cultured on coated culture dishes. As coated culture dishes, use can be made of, for example, culture dishes coated with Matrigel, collagen, gelatin, laminin, and fibronectin. Preferably, Matrigel-coated culture dishes are used.

By way of example, the basal medium that can be used in the present invention can be, for example, ES medium [40% of Dulbecco's modified Eagle's medium (DMEM), 40% of F12 medium (manufactured by Sigma), 2 mM of L-glutamine or GlutaMAX (manufactured by Sigma), 1% of non-essential amino acids (manufactured by Sigma), 0.1 mM of β-mercaptoethanol (manufactured by Sigma), 15 to 20% of Knockout Serum Replacement (manufactured by Invitrogen), 10 µg/ml of gentamicin (manufactured by Invitrogen), 4 to 10 ng/ml of bFGF (FGF2)] (hereinafter referred to as ES medium); a conditioned medium which is a supernatant obtained by culturing mouse embryonic fibroblasts (MEFs) for 24 hours in ES medium without 0.1 mM β-mercaptoethanol and to which are added 0.1 mM of β-mercaptoethanol and 10 ng/ml of bFGF (FGF2) (hereinafter referred to as MEF-conditioned ES medium); an optimized medium for iPS cells (manufactured by iPSellon), an optimized medium for feeder cells (manufactured by iPSellon); StemPro® hESC SFM (manufactured by Invitrogen), mTeSR1 (manufactured by STEMCELL Technology Veritas); an animal protein-free, serum-free medium for maintenance of human ES and iPS cells, TeSR2 [ST-05860] (manufactured by STEMCELL Technology Veritas); a medium for primate ES and iPS cells (REPROCELL Inc.); ReproStem (REPROCELL Inc.); ReproFF (REPROCELL Inc.); and ReproFF2 (REPROCELL Inc.), without limitation thereto. If the basal medium to be used does not contain one or more of serum, A-83-01, and CHIR99021, which are required in the preparation method of the present invention, then the medium should be added with the one or more of serum, A-83-01, and CHIR99021 as appropriate.

In the present invention, culture conditions optimal for preparation of human liver progenitor cells from human mature hepatocytes can be modified as appropriate, according to routine procedures. Culture conditions optimal for preparation of human liver progenitor cells from human mature hepatocytes include, but are limited to, the following:
For culture temperature, 15°C to 45°C is preferable, 25°C to 40°C is further preferable, and 37°C is most preferable.

For CO₂ concentration, 1% to 10% is preferable, 3% to 8% is further preferable, and 5% is most preferable.

For culture period, a period of 1 day (24 hours) to 30 days is preferable, and a period of 3 days to 20 days is further preferable.

In the present invention, procedures for expansion or passage culturing of the liver progenitor cells can use any of methods usually used by those skilled in the art in culturing ES cells, iPS cells, and others. Such methods can include, by way of example, a method by which passage culturing of the liver progenitor cells are carried out, for example, by removing the medium from the culture dish on which the liver progenitor cells have been cultured, washing them with PBS(-), and adding a cell detaching solution to the culture dish and allowing the culture dish to stand, and then adding D-MEM (high glucose) medium containing 1 × antibiotic-antifungal agent and 10% FBS, followed by centrifugation and removing the supernatant, followed by adding 1 × antibiotic-antifungal agent, mTeSR1, and 10 µM Y-27632 to prepare a cell suspension, which is then seeded on a Matrigel-, gelatin-, or collagen-coated culture dish on which MEF cells have been seeded.

The liver progenitor cells in the present invention can be of any type, as long as they are capable of differentiation into mature hepatocytes, biliary epithelial cells, and others. The liver progenitor cells may be those that have been subjected to freeze storage, or those that have been repeatedly subjected to freeze storage and thawing as appropriate. In the present invention, freeze storage can be performed by suspending the liver progenitor cells in a cryopreservation solution well-known to those skilled in the art and cooling the cell suspension. The suspension can be made by detaching the cells from the culture dish with a detaching agent such as trypsin, transferring them into cryopreservation containers, treating them as appropriate, and then adding a cryopreservation solution into the cryopreservation containers.

The cryopreservation solution may contain DMSO (Dimethyl sulfoxide) as a cryoprotectant. However, DMSO is cytotoxic, and thus the content thereof is preferably reduced. In an embodiment, DMSO is substituted with, for example, glycerol, a propylene glycol, or a polysaccharide. When DMSO is used as a cryoprotectant, DMSO is contained at a concentration of 5% to 20%, preferably 5% to 10%, more preferably 10%. Besides these, cryopreservation solutions may comprise additives described in WO2007/058308. These cryopreservation solutions that may be used in present invention include ones available from, for example, BioVerde, Inc.; Nippon Genetics Co., Ltd.; REPROCELL Inc.; ZENOAQ; Cosmo Bio Co. Ltd.; Kojin-Bio Co., Ltd.; and Thermo Fisher Scientific Inc.

When the liver progenitor cells that have been suspended as described above are subjected to freeze storage, the cells are suitably stored at a temperature between -80°C and -100°C (for example, -80°C), which can be carried out using any freezer allowing this temperature to be reached. In this case, without particular limitation, a programmable freezer can be used to regulate the cooling rate as appropriate, in order to avoid rapid temperature changes. The rate at which the cells are cooled can be appropriately selected depending on the components of a cryopreservation solution, and the cooling of the cells can be performed according to the manufacturer's instruction of the cryopreservation solution.

The period for which the liver progenitor cells are subjected to freeze storage, especially the upper limit thereof, is not limited, as long as the cells that have been thawed after freeze storage under the above-described conditions retain properties equivalent to those of the liver progenitor cells prior to freeze storage, and includes, for example, 1 week or more, 2 weeks or more, 3 weeks or more, 4 weeks or more, 2 months or more, 3 months or more, 4 months or more, 5 months or more, 6 months or more, 1 year or more, or more than these. Storage at lower temperatures allows inhibition of cell damage, and thus the liver progenitor cells can be transferred and stored in vapor phase above liquid nitrogen (at a temperature of not more than about -150°C to not more than -180°C). When the liver progenitor cells are stored in vapor phase above liquid nitrogen, the storing can be carried out using storage containers well-known to those skilled in the art. Without particular limitation, by way of example, when the liver progenitor cells are subjected to freeze storage for a period of two weeks or more, the cells are preferably stored in vapor phase above liquid nitrogen.

The liver progenitor cells that have been subjected to freeze storage can be thawed by methods well-known to those skilled in the art. By way of example, the thawing is done, for example, by a method of thawing by which the frozen liver progenitor cells are left to stand or shaken in an incubator or water bath at 37°C.

The liver progenitor cells obtained by the present invention can be further cultured, thereby to allow their differentiation into mature hepatocytes, biliary epithelial cells, or the like.

When compared with the liver progenitor cells, the mature hepatocytes thus obtained (hereinafter referred to as "differentiated mature hepatocytes") result in a significantly increased amount of expression of at least one gene selected from the group consisting of the following genes: ALB (Albumin), AFP (Alpha Fetoprotein), TAT (Tyrosine Aminotransferase), TDO2 (Tryptophan 2,3-dioxygenase), TTR (Transthyretin), G6PC (Glucose-6-phosphatase), NTCP (sodium taurocholate cotransporting polypeptide), Cnx32, CYP1A1 (Cytochrome P1A1), CYP1A2 (Cytochrome P1A2), CYP2B6 (Cytochrome P2B6), CYP2C9 (Cytochrome P2C9), CYP2C19 (Cytochrome P2C19), CYP2D6 (Cytochrome P2D6), CYP3A4 (Cytochrome P3A4), and CYP7A1 (Cytochrome P7A1) genes. Without particular limitation, the differentiated mature hepatocytes are characterized in that the expression of the at least one gene is increased to an amount 10 to 50,000 times higher than that provided by the liver progenitor cells.

Differentiation induction (also referred to hereinafter as "differentiation" or "induction") from the human liver progenitor cells into differentiated mature hepatocytes can be carried out, for example, by adding oncostatin M and dexamethasone to a basal medium, such as a basal medium as described above. The amount of oncostatin M required to provide the concentrations of respective growth differentiation factors in the differentiation induction medium is, for example, from 1 µg to 100 µg, preferably from 5 µg to 50 µg, per liter of the medium. The amount of dexamethasone required to provide the concentrations of respective growth differentiation factors in the differentiation induction medium is, for example, from 0.1 mM to 10 mM, preferably from 0.5 mM to 5 mM, per liter of the medium.

Differentiation induction from the human liver progenitor cells into differentiated mature hepatocytes can also be carried out by transplanting the human liver progenitor cells into the liver or spleen of an animal. The animal into which the liver progenitor cell are to be transplanted is not limited in particular, as long as the animal is capable of inducing the differentiation of the human liver progenitor cells into differentiated mature hepatocytes, and is desirably a mammal, such as a rabbit, a dog, a cat, a guinea pig, a hamster, a mouse, a rat, a sheep, a goat, a pig, a minipig, a horse, a cow, and a simian, with a mouse, a rat, a minipig, and a simian being further preferred.

The mature hepatocytes that have been differentiated from the liver progenitor cells (differentiated mature hepatocytes) have, for example, the ability to produce glucose, metabolize ammonia, produce albumin, and synthesize urea. The ability to produce glucose can be confirmed, for example, by analyzing the level of glucose in a cultured supernatant from the differentiated mature hepatocytes by means of a glucose oxidase method. The ability to metabolize ammonia can be confirmed, for example, by analyzing the level of ammonia in a cultured medium from the differentiated mature hepatocytes by means of a modified indophenol method (Horn DB & Squire CR, Chim. Acta., 14: 185-194, 1966). The ability to produce albumin can be confirmed, for example, by analyzing the concentration of albumin in a cultured solution from the differentiated mature hepatocytes by means of a method for measuring the serum albumin concentration. The ability to synthesize urea can be confirmed, for example, by using Colorimetric assay (Sigma).

The present invention can provide liver progenitor cells produced by the method of the present invention, and mature hepatocytes differentiated therefrom (differentiated mature hepatocytes). The liver progenitor cells and mature hepatocytes differentiated therefrom (differentiated mature hepatocytes) are characterized by being more similar in function and morphology to human mature hepatocytes than to hepatocytes produced by conventional methods. In addition, the liver progenitor cells and mature hepatocytes differentiated therefrom (differentiated mature hepatocytes) are characterized by being also functional in vivo. Therefore, the liver progenitor cells and mature hepatocytes differentiated therefrom (differentiated mature hepatocytes) of the present invention are useful, for example, in the field of medicine, such as regenerative medicine.

For example, liver diseases can be treated by using the liver progenitor cells of the present invention. Liver diseases can be treated, for example, with methods by which the liver progenitor cells or mature hepatocytes differentiated therefrom (differentiated mature hepatocytes) are transplanted into the liver directly through the hepatic portal vein or transplanted in forms in which the liver progenitor cells or differentiated mature hepatocytes have been entrapped in collagen, polyurethane, or other known biocompatible materials. Thus, the present invention also provides the use of liver progenitor cells produced by the above process or mature hepatocytes differentiated therefrom (differentiated mature hepatocytes). More specifically, the present invention provides a therapeutic agent for a liver disease, the therapeutic agent comprising the liver progenitor cells of the present invention or mature hepatocytes differentiated therefrom (differentiated mature hepatocytes). The present invention also provides a method for treating a liver disease using the liver progenitor cells of the present invention or mature hepatocytes differentiated therefrom (differentiated mature hepatocytes). Specific examples of the liver disease that can be treated by the method of the present invention include, but are not limited to, liver cirrhosis, fulminant hepatitis, chronic hepatitis, viral hepatitis, alcoholic hepatitis, hepatic fibrosis, autoimmune hepatitis, fatty liver, drug-induced allergic liver injury, hemochromatosis, hemosiderosis, Wilson's disease, primary biliary cirrhosis, primary sclerosing cholangitis, liver abscess, chronic active hepatitis, chronic persistent hepatitis biliary atresia, liver cancer, and others.

Similarly to the differentiated mature hepatocytes, the biliary epithelial cells that have been differentiated from the liver progenitor cells can be confirmed, for example, on the basis of their morphological features and epithelial cell markers.

The present invention can also provide biliary epithelial cells that have been differentiated from the liver progenitor cells produced by the method of the present invention (which are hereinafter referred to as "differentiated biliary epithelial cells"). The biliary epithelial cells produced by the method of the present invention are characterized by being more similar in function and morphology to human biliary epithelial cells than to biliary epithelial cells produced by conventional methods. In addition, the differentiated biliary epithelial cells are characterized by being also functional in vivo. Therefore, the liver progenitor cells and biliary epithelial cells differentiated therefrom (differentiated biliary epithelial cells) of the present invention are useful, for example, in the field of medicine, such as regenerative medicine.

For example, the use of the liver progenitor cells of the present invention allows the use of the liver progenitor cells or biliary epithelial cells of the present invention for the treatment of bile duct diseases, such as gallstones, gallbladder polyps, gallbladder cancer, cholangiocarcinoma, cholangitis, cholangiohepatitis, cholecystitis, Alagille syndrome (AGS), gallbladder stones, gallbladder adenomyomatosis, polypoid lesions of the gallbladder, acalculous biliary pain, and primary sclerosing cholangitis (PSC).

The liver progenitor cells of the present invention are also useful, for example, in a research field aiming at treating liver diseases. For example, the liver progenitor cells can be used in the research and development of artificial organs, such as artificial liver. Furthermore, the human hepatocytes of the present invention are also useful in the field of development of pharmaceutical products, foods, and others. Specifically, the human hepatocytes can be utilized for evaluation of the metabolism and hepatotoxicity of test substances, and for screening of therapeutic agents for liver diseases, inhibitors against hepatitis virus infection, or therapeutic agents for viral hepatitis.

The metabolism and hepatotoxicity of test substances can be evaluated by utilizing liver progenitor cells produced by the method of the present invention, or mature hepatocytes differentiated therefrom (differentiated mature hepatocytes) or biliary epithelial cells differentiated therefrom (differentiated biliary epithelial cells).

In the past, animal models and others have been used for evaluation of the metabolism and hepatotoxicity of test substances. However, there is a limitation on the number of test substances that can be evaluated at a time, and there has been caused a problem that the evaluation results obtained using animal models and others are not applicable to humans without any correction. For these reasons, evaluation methods are being adopted which employ human liver cancer cell lines or cultured primary normal human hepatocytes. However, since human liver cancer cell lines are derived from hepatoma cells, a possibility remains that the evaluation results obtained using a human liver cancer cell line are not applicable to human normal hepatocytes. Cultured primary normal human hepatocytes, on the other hand, have problems in terms of stable supply and cost. In addition, cells of a cell line obtained by immortalizing cultured primary normal human hepatocytes have been shown to have decreased activity of CYP3A4, compared to unimmortalized cells (International Journal of Molecular Medicine 14:663-668, 2004, Akiyama I., et al.). These problems can be solved by utilizing liver progenitor cells produced by the method of the present invention, or mature hepatocytes differentiated therefrom (differentiated mature hepatocytes) or biliary epithelial cells differentiated therefrom (differentiated biliary epithelial cells).

The differentiated mature hepatocytes of the present invention can be used for screening of test substances. In the present invention, screening of test substances can be carried out, for example, based on analyses of metabolism enzymes, pathways, products, or activities, cytotoxicity, genotoxicity, carcinogenic development, mutagenicity, hepatotoxic development, or hepatic effects for the test substances.

The present invention provides a method for evaluating the metabolism of a test substance. In the method, a test substance is contacted with liver progenitor cells produced by the method of the present invention or mature hepatocytes differentiated therefrom (differentiated mature hepatocytes). Then, a determination is made as to whether the metabolism of the test substance contacted with the liver progenitor cells or differentiated mature hepatocytes takes place.

The test substance for use in the present invention is not limited in particular. For example, the test substance includes, but is not limited to, a single compound such as a xenobiotic, a natural compound, an organic compound, an inorganic compound, a protein, and a peptide; and also a compound library; expression products from a gene library; a cell extract; a cell culture supernatant; microbial fermentation products; marine organism extracts; plant extracts; pharmaceutical raw materials; cosmetic raw materials; food raw materials; pharmaceutical additives; cosmetic additives; food additives; and supplement components.

The xenobiotic can be, for example, a candidate compound or substance of a drug and food, and an existing drug and food, without limitation thereto. The xenobiotic of the present invention includes any material, as long as it is a material foreign to the living body. More specifically, by way of example, the xenobiotics can be Rifampin, Dexamethasone, Phenobarbital, Ciglirazone, Phenytoin, Efavirenz, Simvastatin, β-Naphthoflavone, Omeprazole, Clotrimazole, 3-Methylcholanthrene, and others.

In the present invention, "contacted" or "contacting" is usually carried out by a method of adding a test substance to a medium or a culture solution in which the liver progenitor cells or differentiated mature hepatocytes are cultured, without limitation thereto. When the test substance is a protein or the like, the test substance can be "contacted" by introducing a DNA vector expressing the protein into the liver progenitor cells or differentiated mature hepatocytes.

The metabolism of a test substance can be determined by methods well-known to those skilled in the art. For example, a test substance is judged to be metabolized if a metabolite of the test substance is detected. Also, a test substance is judged to be metabolized if contacting the test substance with the liver progenitor cells or differentiated mature hepatocytes results in the induction of expression of enzyme genes such as CYP (cytochrome p450), MDR (MultiDrug Resistance, ABCB1), and MPR (Multidrug Resistance-Associated Protein, ABCC2), or leads to increased activities of these enzymes.

For analysis of metabolism enzymes, an enzyme involved in the metabolism of a test substance can be analyzed, for example, by analyzing structural changes of the test substance after contacting it with differentiated mature hepatocytes of the present invention. Specifically, the analysis can include, for example, the identification of an enzyme involved in the metabolism of a test substance by analyzing structural changes of the test substance after contacting it with differentiated mature hepatocytes of the present invention, using inhibitors/antagonists of or neutralizing antibodies against various enzymes; an analysis of the enzymatic reaction mechanism for the test substance by analyzing structural changes of the test substance resulting from contacting it with the differentiated mature hepatocytes; and an analysis of the substrate specificity of an enzyme involved in the metabolism of the test substance.

For analysis of metabolism pathways and metabolites, the pathway metabolizing a test substance and a metabolite(s) thereof can be analyzed, for example, by analyzing structural changes of the test substance after contacting it with differentiated mature hepatocytes of the present invention. Known methods can be used as a method for detecting a metabolite(s) of a test substance. By way of example, a metabolite(s) of a test substance can be detected by analyzing a cultured medium or the like obtained from the differentiated mature hepatocytes contacted with the test substance, for example, with liquid chromatography or mass spectrometry.

For analysis of metabolism activities, whether the activity of an enzyme metabolizing a test substance is enhanced can be analyzed, for example, by contacting the test substance with differentiated mature hepatocytes of the present invention, and then detecting an increase in the activity or amount of the metabolizing enzyme, or an increase in the amount of transcription of the gene coding therefor. Specifically, the analysis can be performed by detecting an increase in the enzymatic activity, protein amount, or mRNA amount of the enzyme cytochrome P450. Known methods can be used as a detection method for this purpose, including measurement of the activities of various P450 isoform enzymes, western blotting for various P450 isoform proteins, Northern hybridization or RT-PCR methods for various P450 isoform mRNAs, and others.

The present invention also provides a method for evaluating the hepatotoxicity of a test substance. In the method, a test substance is contacted with liver progenitor cells produced by the method of the present invention and mature hepatocytes differentiated therefrom (differentiated mature hepatocytes). Then, the degree of damage to the liver progenitor cells or differentiated mature hepatocytes contacted with the test substance is determined. The extent of their damage can be determined, for example, using as an index, their survival rate or liver injury markers such as GOT (glutamate oxaloacetate transaminase) and GPT (glutamic pyruvic transaminase).

For analysis of hepatotoxic development, the hepatotoxicity resulting from the metabolism of a test substance can be analyzed, for example, by contacting the test substance with differentiated mature hepatocytes of the present invention, and observing the development of toxic effects on the differentiated mature hepatocytes, or alternatively by contacting the test substance with the differentiated mature hepatocytes, and then administering the test substance that have been modified by the differentiated mature hepatocytes to other hepatocytes, liver sections, excised liver, or experimental animals, followed by observing resultant changes in these cells, tissues, or living bodies.

For example, a test substance is judged to be hepatotoxic if addition of the test substance to a culture solution for the liver progenitor cells or mature hepatocytes derived therefrom (differentiated mature hepatocytes) leads to a reduction in their survival rate, and is judged not to be hepatotoxic if the addition does not lead to a significant change in their survival rate. Also, a test substance is judged to be hepatotoxic, for example, if addition of the test substance to a culture solution for the liver progenitor cells or differentiated mature hepatocytes leads to an increase in the level of GOT or GPT in the cultured solution, and is judged not to be hepatotoxic if the addition does not lead to a significantly change in the level of GOT or GPT in the cultured solution.

In these cases, whether or not a test substance is hepatotoxic can be more accurately evaluated when a substance that has already been determined to be hepatotoxic is used as a reference.

For analysis of cytotoxicity, the cytotoxicity resulting from a metabolite(s) of a test substance can be analyzed, for example by contacting the test substance with differentiated mature hepatocytes of the present invention. Specifically, the analysis is performed by observing, for example, morphological changes, variation in the number of viable cells, leakage of intracellular enzymes, structural changes in the cell surface layer, or changes in intracellular enzymes of the differentiated mature hepatocytes due to contacting the test substance with them.

For analysis of genotoxicity, the genotoxicity resulting from the metabolism of a test substance can be analyzed, for example, by contacting the test substance with differentiated mature hepatocytes of the present invention and subjecting the differentiated mature hepatocytes to a chromosomal aberration test, a micronucleus test, or the like. Also, the analysis can be performed by contacting the test substance with differentiated mature hepatocytes of the present invention, and then subjecting the differentiated mature hepatocytes to a chromosomal aberration test, a micronucleus test, a reverse mutation test, or the like by evaluating in a suitable evaluation system the test substance that has been modified by the differentiated mature hepatocytes.

For analysis of carcinogenic development, the carcinogenicity resulting from the metabolism of a test substance can be analyzed, for example, by contacting the test substance with differentiated mature hepatocytes of the present invention and subjecting the differentiated mature hepatocytes to a chromosomal aberration test, DNA modification, or the like. Also, the carcinogenicity can be analyzed by contacting the test substance with differentiated mature hepatocytes of the present invention, and then evaluating the test substance that has been modified by the differentiated mature hepatocytes, in a suitable carcinogenesis evaluation system with a suitable chemical substance.

For analysis of mutagenicity, the mutagenicity resulting from the metabolism of a test substance can be analyzed, for example, by contacting the test substance with differentiated mature hepatocytes of the present invention and subjecting the differentiated mature hepatocytes to a chromosomal aberration test, a micronucleus test, or the like. Also, the analysis can be performed by contacting the test substance with differentiated mature hepatocytes of the present invention, and then subjecting the differentiated mature hepatocytes to a chromosomal aberration test, a micronucleus test, a reverse mutation test, or the like by evaluating in a suitable evaluation system the test substance that has been modified by the differentiated mature hepatocytes.

The present invention can also provide a method for screening therapeutic agents for a liver or bile duct disease. In the method, a test substance is contacted with liver progenitor cells produced by the method of the present invention or mature hepatocytes differentiated therefrom (differentiated mature hepatocytes). Then, the function of the liver progenitor cells or differentiated mature hepatocytes that have been contacted with the test substance is determined. Then, a substance is selected which results in enhanced function of the liver progenitor cells or differentiated mature hepatocytes that have been contacted with the test substance.

For analysis of hepatic effects, effects of a test substance on the liver can be analyzed, for example, by contacting the test substance with differentiated mature hepatocytes of the present invention, and then observing resultant changes in the differentiated mature hepatocytes, or alternatively by contacting the test substance with the differentiated mature hepatocytes, and then administering the test substance that have been modified by the differentiated mature hepatocytes to other hepatocytes, liver sections, excised liver, or experimental animals, followed by observing resultant changes in these cells, tissues, or living bodies. In this case, it is expected that a test substance will have a therapeutic effect on the liver when enhanced cell function is found in the differentiated mature hepatocytes that have been contacted with the test substance.

In the present invention, the function of the liver progenitor cells or mature hepatocytes differentiated therefrom (differentiated mature hepatocytes) can be determined, for example, using as an index the ability to produce glucose, metabolize ammonia, produce albumin, and synthesize urea, and the activities of enzymes such as CYPs.

The ability to produce glucose can be confirmed, for example, by analyzing the level of glucose in a cultured supernatant from the liver progenitor cells or differentiated mature hepatocytes by means of a glucose oxidase method. The ability to metabolize ammonia can be confirmed, for example, by analyzing the level of ammonia in a cultured medium from the liver progenitor cells or differentiated mature hepatocytes by means of a modified indophenol method (Horn DB & Squire CR, Chim. Acta., 14: 185-194, 1966). The ability to produce albumin can be confirmed, for example, by analyzing the concentration of albumin in a cultured solution from the liver progenitor cells or differentiated mature hepatocytes by means of a method for measuring the serum albumin concentration. The ability to synthesize urea can be confirmed, for example, by using Colorimetric assay (Sigma). The CYP in the present invention is not limited in particular, and includes, for example, CYP1A1, CYP2C8, CYP2C9, and CYP3A4. Method for measuring the activities of these CYP isoforms can use those well-known to those skilled in the art.

Liver progenitor cells produced by the method of the present invention and mature hepatocytes differentiated therefrom (differentiated mature hepatocytes) are allowed to be infected with a hepatitis virus because the function and morphology of the liver progenitor cells or differentiated mature hepatocytes are more similar to those of human mature hepatocytes.

The present invention provides a method for screening inhibitors against hepatitis virus infection. In the method, a hepatitis virus is contacted with liver progenitor cells produced by the method of the present invention or mature hepatocytes differentiated therefrom (differentiated mature hepatocytes), in the presence of a test substance. Then, an examination is made as to the presence or absence of hepatitis virus infection in the liver progenitor cells or differentiated mature hepatocytes contacted with the hepatitis virus. Then, a substance inhibiting the hepatitis virus infection is selected. The contacting of the hepatitis virus to the liver progenitor cells or differentiated mature hepatocytes can be carried out by routine procedures.

The hepatitis virus includes, without particular limitation, hepatitis C virus, hepatitis A virus, and hepatitis B virus. These hepatitis viruses may be established virus strains, or isolates obtained directly from hepatitis virus infected individuals. The hepatitis virus may be in a purified form or in a crude form (for example, in the form of serum obtained from an infected individual).

The presence or absence of hepatitis virus infection can be confirmed, for example, based on the amount of hepatitis virus in the liver progenitor cells or differentiated mature hepatocytes. The amount of hepatitis virus therein can be determined, for example, based on the amount of hepatitis virus RNA therein. The amount of hepatitis virus RNA can be measured according to routine procedures, or by a method established by the present inventors (T. Takeuchi et al., Real-Time Detection System for Quantification of Hepatitis C Virus Genome. Gastroenterology 1999, 116:636-642).

Furthermore, the present invention can provide a method for screening therapeutic agents for viral hepatitis. In the method, a hepatitis virus is contacted with liver progenitor cells produced by the method of the present invention or mature hepatocytes differentiated therefrom (differentiated mature hepatocytes). Then, a test substance is contacted with the liver progenitor cells or differentiated mature hepatocytes that have been infected with the hepatitis virus. Then, the propagation of the hepatitis virus therein is determined. Then, a substance is selected which inhibits the propagation of the hepatitis virus.

Examples of a substance that inhibits the propagation of hepatitis virus include all of the following: 1) a substance that inhibits the propagation of hepatitis virus, compared with when the test substance is not brought in contact with the infected liver progenitor cells or differentiated mature hepatocytes, 2) a substance that completely inhibits the propagation of hepatitis virus, and 3) a substance that eliminates hepatitis virus. The propagation or disappearance of hepatitis virus can be confirmed by measuring the amount of hepatitis virus in the liver progenitor cells of differentiated mature hepatocytes.

In the present invention, a test substance can usually be contacted with the differentiated mature hepatocytes by adding the test substance to a medium or culture solution for the differentiated mature hepatocytes. Otherwise, a test substance can be contacted with the differentiated mature hepatocytes by expressing a gene encoding the test substance in the differentiated mature hepatocyte. Alternatively, a test substance can be contacted with the differentiated mature hepatocytes by co-culturing them together with cells producing the test substance.

The differentiated mature hepatocytes of the present invention are more similar in function and morphology to human mature hepatocytes, and thus can be infected with a hepatitis virus. Therefore, the present invention can provide a method for culturing a hepatitis virus. In the method for culturing a hepatitis virus according to the present invention, the type of hepatitis virus that is to be cultured is not limited in particular, and includes, for example, hepatitis A virus, hepatitis B virus, hepatitis C virus, hepatitis D virus, and hepatitis E virus. The culturing method of the present invention is useful for passage and propagation of already isolated hepatitis viruses. The culturing method of the present invention can also allow isolation of hepatitis viruses from samples taken from the environment or samples obtained from patients.

In an embodiment, the present invention provides a model animal of human liver. The model animal of human liver of the present invention is obtained by transplanting differentiated mature hepatocytes of the present invention into a non-human mammal. Routes of administration of the differentiated mature hepatocytes for their transplantation into a non-human mammal include, for example, administration directly onto the surface of the liver, intrahepatic administration, intraportal administration, intrasplenic administration, oral administration, subcutaneous administration, intramuscular administration, intravenous administration, intraarterial administration, sublingual administration, rectal administration, vaginal administration, and transdermal administration. In terms of the engraftment rate of the differentiated mature hepatocytes, preference is given to administration directly onto the surface of the liver, intrahepatic administration, intrasplenic administration, intraarterial administration, and intravenous administration, with intrahepatic administration, intrasplenic administration, and intrahepatic arterial administration being more preferable.

In the production of the model animal of human liver of the present invention, the dosage of the differentiated mature hepatocytes may vary depending on the non-human mammal and route of administration, and is usually from 1 × 10 to 1 × 10¹⁰ cells/individual, preferably from 1 × 10² to 1 × 10⁹ cells/individual, further preferably from 1 × 10³ to 1 × 10⁸ cells/individual. Note that these dosages can be administered twice or more times in single doses, or in multiple divided doses.

The non-human mammal for use in the present invention is not limited in particular, as long as it is a mammal, and includes, for example, a rabbit, a dog, a cat, a guinea pig, a hamster, a mouse, a rat, a sheep, a goat, a pig, a minipig, a horse, a cow, a simian, with a mouse, a rat, a minipig, and a simian being further preferable.

Furthermore, the present invention can provide a kit comprising human liver progenitor cells or mature hepatocytes derived therefrom (differentiated mature hepatocytes), wherein the human liver progenitor cells are prepared by culturing human mature hepatocytes in a medium containing serum, A-83-01, and CHIR99021. In an embodiment, such a kit is used for evaluating the metabolism and/or hepatotoxicity of a test substance using the human liver progenitor cells or differentiated mature hepatocytes. In another embodiment, such a kit can be used for screening test substances, for example, particularly for screening therapeutic agents for liver or bile duct diseases, screening inhibitors against hepatitis virus infection, and screening therapeutic agents for viral hepatitis. The respective specific evaluation and screening methods are as previously described.

### EXAMPLES

The present invention now will be described in detail with reference to working examples and test examples, but is not intended to be limited thereto. In the following, ages in months and years regarding to the cells used are abbreviated as "M" (Month) and "Y" (Year), respectively.

### (Example 1)

A first sample of frozen human hepatocytes (Lot. ID: HC3-14, 45 Y, Male, Caucasian; manufactured by Xenotech) was suspended in a thawing medium (William's E medium (manufactured by Life Technologies, 32551-020), 10% FBS (manufactured by Life Technologies), 10⁻⁴ M insulin (manufactured by Sigma), 1 × antibiotic/antimycotic solution (manufactured by Life Technologies), and the human hepatocytes were collected by low speed centrifugation at 500 rpm (about 40 × g) at 4°C for 2 min. The collected hepatocytes were resuspended in a seeding medium (L-15 medium (manufactured by Life Technologies, 11415-064), 1 × antibiotic/antimycotic solution (manufactured by Life Technologies)) to count the number of hepatocytes. The cell suspension was seeded in collagen-coated plates (manufactured by IWAKI) to give a cell density of 1 × 10⁴ cells/cm², and placed into a CO₂ incubator (37°C, 5% CO₂). At 3 to 5 hours after the cells adhered onto the plate, the medium was changed from the seeding medium to one of three testing media: AC-F medium in which 10% FBS (manufactured by Life Technologies), 0.5 µM A-83-01 (manufactured by WAKO), and 3 µM CHIR99021 (manufactured by Axon Medchem) were added to basal medium (hereinafter referred to as "SHM medium" [DMEM/F12 medium (manufactured by Life Technologies, 11320033), 5 mM HEPES (manufactured by Sigma, St. Louis, MO), 30 mg/L L-proline (manufactured by Sigma), 0.05% BSA (manufactured by Sigma), 10 ng/mL epidermal growth factor (manufactured by Sigma), insulin-transferrin-serine (ITS)-X (manufactured by Life Technologies), 10⁻⁷ M dexamethasone (Dex) (manufactured by Sigma), 10 mM nicotinamide (manufactured by Sigma), 100 mM ascorbic acid-2 phosphate (manufactured by Wako), 1 x antibiotic/antimycotic solution (manufactured by Life Technologies]); YAC-F medium in which 10% FBS (manufactured by Life Technologies), 0.5 µM A-83-01 (manufactured by WAKO), 3 µM CHIR99021 (manufactured by Axon Medchem), and 10 µM Y-27632 (manufactured by WAKO) were added to basal medium; and YAC medium in which 0.5 µM A-83-01 (manufactured by WAKO), 3 µM CHIR99021 (manufactured by Axon Medchem), and 10 µM Y-27632 (manufactured by WAKO) were added to basal medium. From then on, the testing media were exchanged for the corresponding fresh testing media at a frequency of once every 2 to 3 days, and culturing was carried out in a CO₂ incubator (37°C, 5% CO₂).

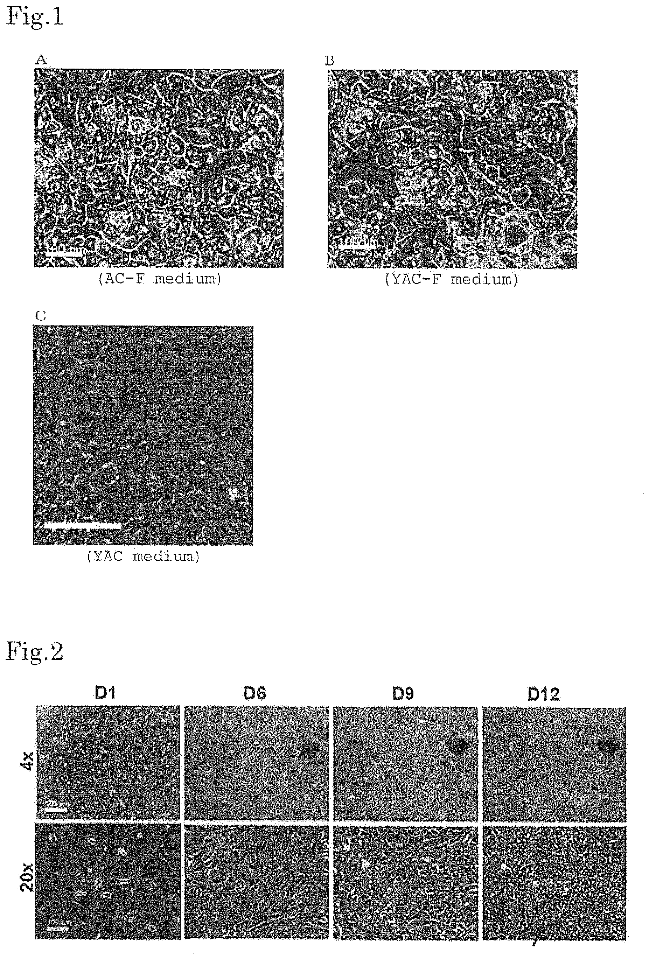

After 17 days of culturing, the morphology of cultured cells was examined under a phase contrast microscope. When the hepatocytes were cultured in AC-F and YAC-F media, there were found many small-sized cells, which had a high Nuclei/Cytoplasm (N/C) ratio and were observed to have a white nucleus, thereby allowing us to confirm that these cells are liver progenitor cells (Figs. 1A and 1B). On the other hand, when the hepatocytes were cultured in YAC medium, there were also found, after 12 days of culturing, cells that had a low N/C ratio,a black nucleus and double nucleus (Fig. 1C), which allowed us to confirm that these cells are not liver progenitor cells. The rate of cell growth was higher when the hepatocytes were cultured in AC-F and YAC-F media than when cultured in YAC medium.

From these results, it turned out that in order to obtain liver progenitor cells from mature hepatocytes, the medium has to contain serum, A-83-01, and CHIR99021.

### (Example 2)

A second sample of frozen human hepatocytes (10 M, Female, Hispanic; manufactured by Celsis) was cultured in AC-F medium as in Example 1. At days 6 (D6), 9 (D9), and 12 (D12) after culturing, there were observed liver progenitor cells (Fig. 2). In Fig. 2, the arrow indicates a cell that resulted from spontaneous differentiation from one of the liver progenitor cell and became mature.

### (Example 3)

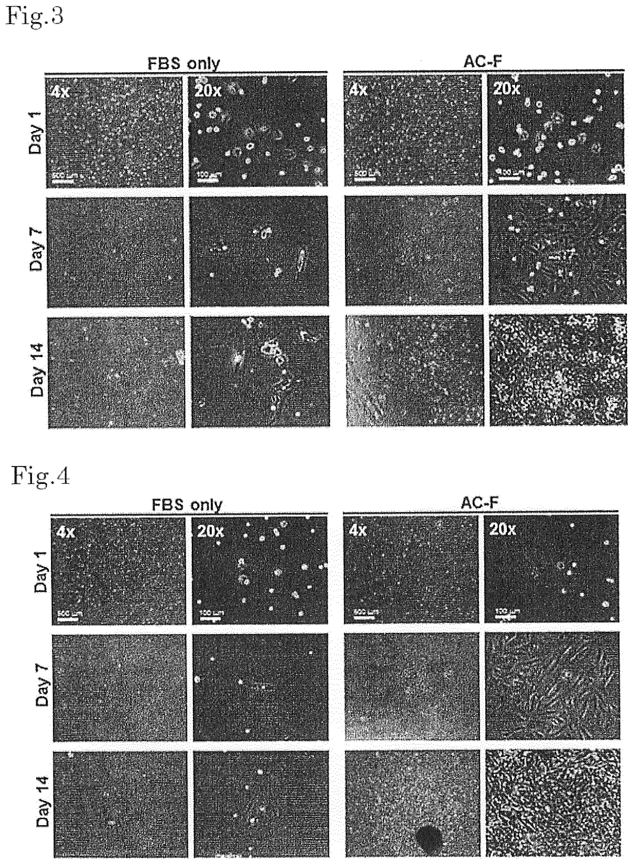

A third sample of frozen human hepatocytes (2Y, Male, Caucasian; manufactured by Biopredic) was cultured in AC-F medium as in Example 1. At days 7 and 14 after culturing, there were observed liver progenitor cells. On the other hand, no liver progenitor cells were observed when the hepatocytes were cultured using, as a testing medium, FBS medium containing only 10% FBS (manufactured by Life Technologies) (Fig. 3).

Likewise, a fourth sample of frozen human hepatocytes (8 M, Male, Caucasian; manufactured by Bioreclamation IVT) was cultured in AC-F medium. At days 7 and 14 after culturing, there were observed liver progenitor cells, whereas no liver progenitor cells were observed when the hepatocytes were cultured using FBS medium as a testing medium (Fig. 4).

### (Example 4)

In this Example, hepatocytes derived from liver progenitor cells of the present invention are transplanted into cDNA-uPA/SCID mice, which develop chronic liver injury due to hepatocyte-specific expression of the uPA gene, resulting in the liver continuing to be congenitally damaged; it is confirmed that the hepatocytes are engrafted in the liver of the cDNA-uPA/SCID mice.

Frozen human hepatocytes (10 M, Female, Hispanic; manufactured by Celsis) were cultured in AC-F medium for 4 days as in Example 1. The cultured cells were washed twice with PBS(-). Then, the cells were detached from the plate using TrypLE Express (manufactured by Thermo, SKU: 12604013) and collected to determine the number of cells. The cell suspension was centrifuged (200 × g, 5 minutes), and the cell pellet was suspended in DMEM10 (10% FBS-DMEM) to make a cell suspension having 5 × 10⁷ cells/mL.

cDNA-uPA/SCID mice (Tateno et. al., 2015; 2 to 4 weeks old) were subjected to laparotomy under isoflurane anesthesia. The spleen was exposed, 0.5 × 10⁵ to 2 × 10⁶ cells per mouse were implanted, and then the abdomen was sutured. Once a week, 20 to 40 uL of blood is collected from the orbit to separate serum. Human-specific albumin in the serum is measured using ALB Human ALB ELISA kit (manufactured by Bethyl, product code: E 88-129). Necropsy is performed 8 weeks after cell implantation to prepare whole blood samples and liver/spleen tissue samples (paraffin-embedded and frozen blocks).

### (Example 5)

In this Example, hepatocytes derived from liver progenitor cells of the present invention are transplanted into TK-NOG mice, in which since thymidine kinase is expressed specifically in hepatocytes, administration of ganciclovir allows induction of hepatocyte-specific cell death, leading to liver injury; it is confirmed that the hepatocytes are engrafted in the liver of the TK-NOG mice.

Frozen human hepatocytes (10 M, Female, Hispanic; manufactured by Celsis) were used to prepare a cell suspension as in Example 4. 10 mg of ganciclovir (GCV; manufactured by Sigma, G2536-100 MG) was weighed, dissolved in 16.7 mL of PBS(-), sterilized by filtration through a 0.22 um filter, and intraperitoneally administered to TK-NOG mice (Hasegawa et. al., 2011; 7 to 8 weeks old; manufactured by In-Vivo Science Inc.) at 10 uL/g body weight (6 mg/kg) at 7 and 5 days prior to cell transplantation. The TK-NOG mice were subjected to laparotomy under isoflurane anesthesia. The spleen was exposed, 0.5 × 10⁵ to 2 × 10⁶ cells per mouse were administered, and then the abdomen was sutured. Once a week, 20 to 40 uL of blood is collected from the tail vein to separate serum. Human-specific albumin in the serum is measured using ALB Human ALB ELISA kit (manufactured by Bethyl, product code: E 88-129). Necropsy is performed 8 weeks after cell administration to prepare whole blood samples and liver/spleen tissue samples (paraffin-embedded and frozen blocks).

### (Example 6)

### Gene expression examinations for confirming the differentiation of liver progenitor cells into hepatocytes

In a similar way as in Example 1, frozen human hepatocytes are cultured in AC-F medium to prepare liver progenitor cells. After that, the liver progenitor cells are cultured for 6 days in a medium containing oncostatin M (OSM) and dexamethasone (Dex), followed by culturing on Matrigel, thereby to allow their differentiation into hepatocytes. For the differentiated hepatocytes, gene expression data is obtained with a one-color microarray-based gene expression analysis system (manufactured by Agilent Technologies), using SurePrint G3 Rat GE 8 x 60K Kit (G4853A) and SurePrint G3 Mouse GE 8 x 60K Ver 2.0 Kit (G4852B) according to their instruction manuals. Intensity values are subjected to logarithmic transformation with base 2, and the date are loaded into Partek Genomics Suite 6.6 (manufactured by Partek Inc., Chesterfield, MO, USA). In the analysis of gene expression, one-way ANOVA is used to identify genes that exhibit differential expression. In the analysis of each of such genes, the P value and the ratio of changes in gene expression are calculated. Using Partek Genomics Suite 6.6, Unsupervised clustering is performed and heat maps are created for all the data sets or for the sorted data sets by the procedure of Euclidean distances of average linkage clustering using the selected probe sets, thereby to confirm the expression of liver-specific genes.

### (Example 7) Examination for confirming the differentiation of liver progenitor cells into biliary epithelial cells Secretin assay

In a similar way as in Example 1, frozen human hepatocytes are cultured in AC-F medium to prepare liver progenitor cells. After that, the liver progenitor cells are cultured on MEFs for 6 days in a medium containing mTeSR1 and YAC, followed by addition of 2% Matrigel and culturing for another 2 days, thereby to allow their differentiation into biliary epithelial cells. The differentiated biliary epithelial cells are cultured for 30 minutes with addition of rat secretin (manufactured by Wako) at 2 × 10⁻⁷ M. After that, the expansion of the lumen region in the bile duct-like structure is examined under a phase contrast microscope to confirm the differentiation into biliary epithelial cells.

### (Example 8)

### Fluorescein diacetate assay

Liver progenitor cells are differentiated into biliary epithelial cells as in Example 7. Fluorescein diacetate is added to the resulting biliary epithelial cells, which are then cultured for 15 minutes. After that, the medium is exchanged for fresh medium. Culturing is continued for another 30 minutes to allow for further transport of degraded fluorescein into the lumen region. After that, the medium is replaced with HBSS(+) and the distribution of fluorescein is examined under a fluorescence microscope to confirm the differentiation of the liver progenitor cells into biliary epithelial cells.

### (Example 9)

### Examination for confirming the expression of liver-specific genes upon differentiation of liver progenitor cells into hepatocytes

Liver progenitor cells are differentiated into hepatocytes as in Example 6. Total RNA is extracted from the resulting hepatocytes using the miRNeasy Mini Kit (manufactured by QIAGEN, Venlo, The Netherlands). Reverse transcription is performed using High-Capacity cDNA Reverse Transcription Kit (manufactured by Life Technologies) according to the instruction manual. The cDNAs prepared are used as templates for PCR which is carried out using Platinum SYBR Green qPCR SuperMix UDG (manufactured by Invitrogen) to confirm the expression of liver-specific genes in the differentiated hepatocytes.

### (Example 10)

Frozen human hepatocytes (10 M, Female, Hispanic; manufactured by Celsis) were cultured in AC-F medium for 11 days as in Example 1. The cultured cells were washed twice with PBS(-). Then, the cells were detached from the plate using TrypLE Express (manufactured by Thermo, SKU: 12604013) and collected to determine the number of cells. The cell suspension was centrifuged (200 × g, 5 minutes), and the cell pellet was suspended in DMEM10 (10% FBS-DMEM) to make a cell suspension having 5 × 10⁷ cells/mL.

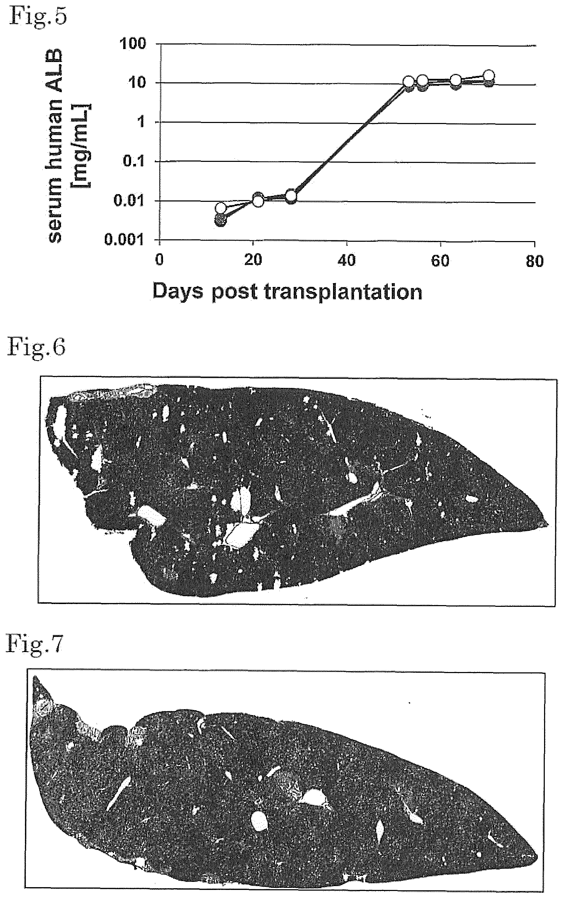

CDNA-uPA/SCID mice (Tateno et. al., 2015; 2 to 4 weeks old, n = 3) were subjected to laparotomy under isoflurane anesthesia. The spleen was exposed, 1 × 10⁶ cells per mouse were implanted, and then the abdomen was sutured. Once a week, 20 to 40 uL of blood was collected from the orbit. Human-specific albumin in blood was measured using ALB Human ALB ELISA kit (manufactured by Bethyl, product code: E 88-129). As shown in Fig. 5, it was able to be confirmed that human ALB was present in the mouse blood, thereby ascertaining that the engraftment of the hepatocytes derived from the liver progenitor cells was achieved. The blood concentrations of human ALB in the respective mice at 70 days after cell administration were 17.2 mg/mL, 12.1 mg/mL, and 12.0 mg/mL.

The livers of these mice were examined for the expression of Human CYP2C9 at 70 days after cell administration. It was confirmed that in the respective mice, Human CYP2C9 was expressed in the liver, in a percent area of 94.6 to 96.1% (in the Right medial lobe), 91.3 to 97.2% (in Left medial lobe), and 93.1 to 96.0% (in Total) (Figs. 6 and 7 for the Right and Left medial lobes, respectively).

### (Example 11)

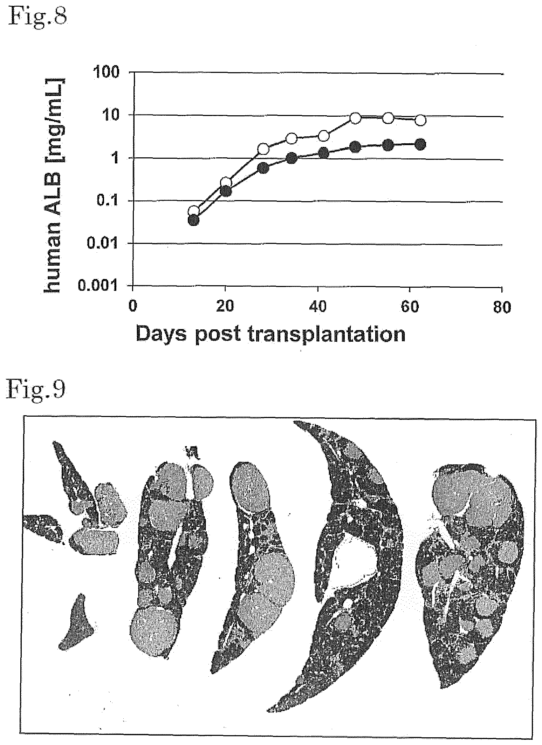

Frozen human hepatocytes (10 M, Female, Hispanic; manufactured by Celsis) were cultured in AC-F medium for 11 days as in Example 10. The cultured cells were washed twice with PBS(-). Then, the cells were detached from the plate using TrypLE Express (manufactured by Thermo, SKU: 12604013) and collected to determine the number of cells. The cell suspension was centrifuged (200 × g, 5 minutes), and the cell pellet was suspended in DMEM10 (10% FBS-DMEM) to make a cell suspension having 5 × 10⁷ cells/mL. Ganciclovir (GCV) was administered to TK-NOG mice (Hasegawa et. al., 2011; 7 to 8 weeks old; manufactured by In-Vivo Science Inc.; n = 2). At 1 week after administration, ALT was measured, and mice with an ALT of 400 to 1600 U/dL were used as recipient animals. For preparing the GCV solution, 500 mg GCV (Mitsubishi Tanabe Pharma Corporation; DENOSINE for I. V. Infusion) was dissolved in 10 mL of water for injection (Otsuka) (at 50 mg/mL), and 0.2 mL aliquots were used as original stock. At the time of transplantation, a working solution was prepared by five-fold dilution of the aliquot with PBS(-). The solution was intraperitoneally administered at 0.1 mL per 10 g of mouse body weight to induce hepatocyte-specific cell death. The recipient TK-NOG mice were subjected to laparotomy under isoflurane anesthesia. The spleen was exposed, 1 × 10⁶ cells/mouse were administered, and then the abdomen was sutured. Once a week, 20 to 40 uL of blood was collected from the tail vein to separate serum. Human-specific albumin was measured in the serum using ALB Human ALB ELISA kit (manufactured by Bethyl, product code: E 88-129). As shown in Fig. 8, it was able to be confirmed that human ALB was present in the mouse serum, thereby ascertaining that the engraftment of the hepatocytes derived from the liver progenitor cells was achieved. The serum concentrations of human ALB in the respective mice at 60 days after cell administration were 8.1 mg/mL and 2.2 mg/mL.

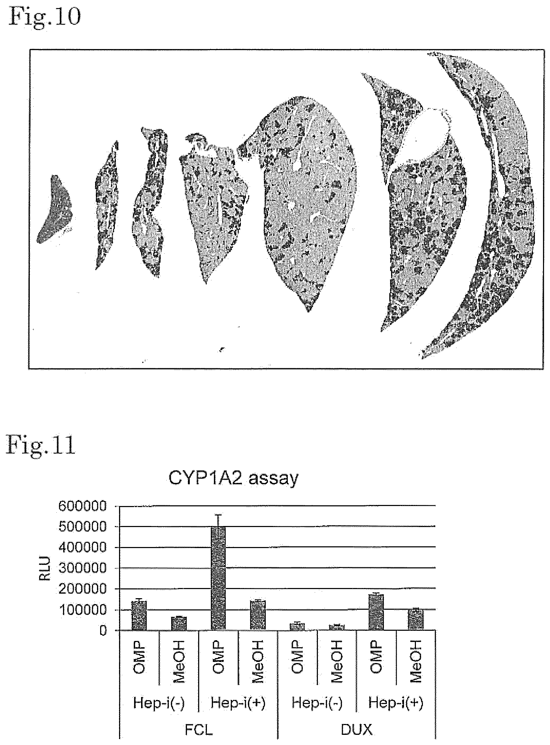

The livers of these mice were examined for the expression of Human CYP2C9 at 60 days after cell administration. It was confirmed that in the respective mice, Human CYP2C9 was expressed in the liver, in a percent area of 57.5% and 30.6% (Figs. 9 and 10).

### (Example 12)

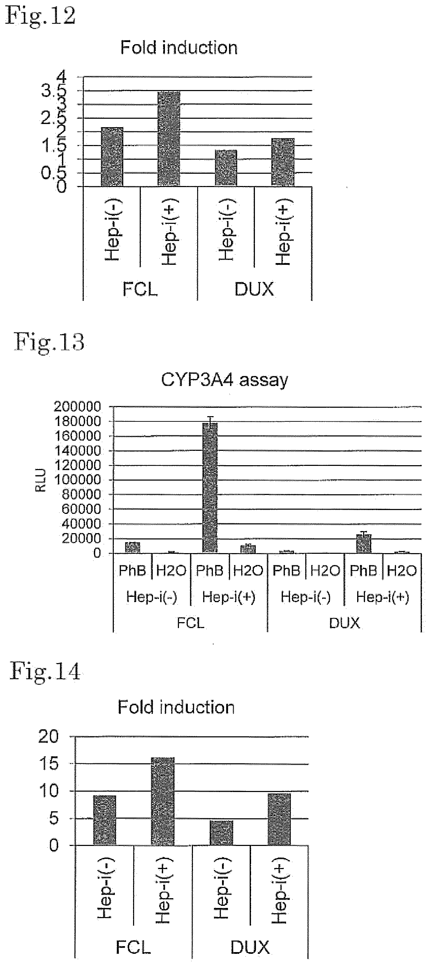

In a similar way as in Example 1, frozen human hepatocytes 1 (10 M, Female, Hispanic; manufactured by Celsis) and frozen human hepatocytes 2 (8 M, Male, Caucasian; manufactured by Bioreclamation IVT) were cultured in AC-F medium to prepare liver progenitor cells (referred to as "FCL" and "DUX", respectively). After that, the liver progenitor cells were cultured for 6 days in a medium containing oncostatin M (OSM, 5 ng/ml) and dexamethasone (Dex, 10⁻⁶ M), followed by culturing on Matrigel for another 2 days, thereby to allow their differentiation into hepatocytes. For both the liver progenitor cells and the hepatocytes differentiated therefrom, the activity of the metabolic enzyme CYP1A2 was measured using methanol (MeOH, at a concentration of 1%) and omeprazole (OMP, 50 µM). In addition, the activity of the metabolic enzyme CYP3A4 was measured using distilled water and phenobarbital (1 mM). The activities of these metabolic enzymes CYP1A2 and CYP3A4 were measured with Luciferin 1A2 kit and Luciferin-IPA kit from Promega, respectively. It was revealed that the differentiation of the liver progenitor cells into hepatocytes lead to the induction of CYP1A2 and CYP3A4 (Figs. 11 to 14).

### (Example 13)

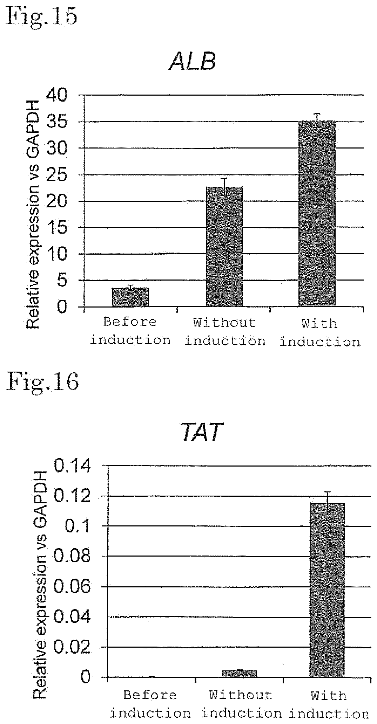

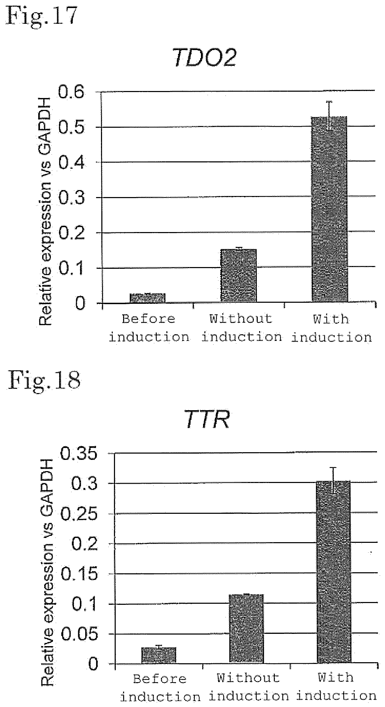

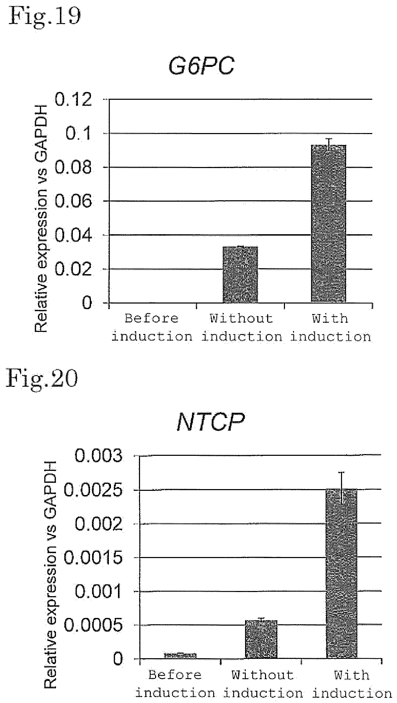

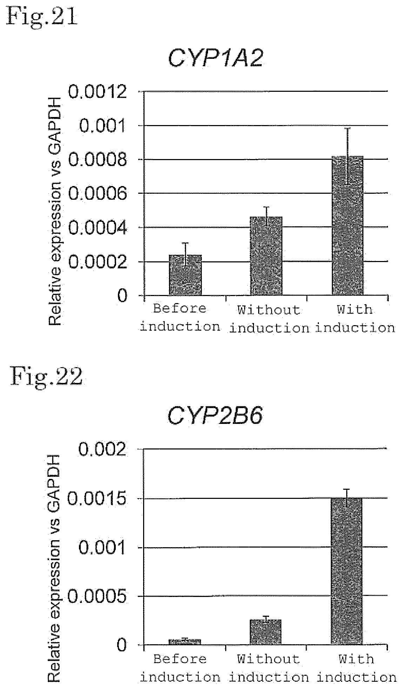

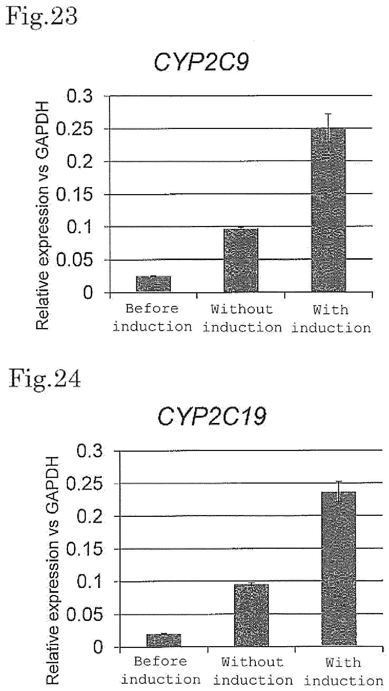

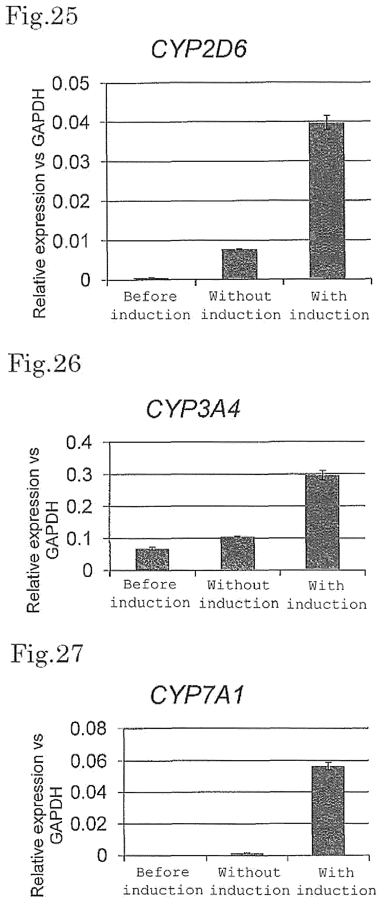

In a similar way as in Example 1, frozen human hepatocytes (10 M, Female, Hispanic; manufactured by Celsis) were cultured in AC-F medium to prepare liver progenitor cells. After that, the liver progenitor cells were cultured for 6 days in a medium containing oncostatin M (OSM, 5 ng/ml) and dexamethasone (Dex, 10⁻⁶ M), followed by culturing on Matrigel for another 2 days, thereby to allow their differentiation into hepatocytes. For both the liver progenitor cells and the hepatocytes differentiated therefrom, the amounts of expression of metabolic enzymes and others were measured using PCR. It was revealed that the differentiation of the liver progenitor cells into hepatocytes lead to the induction of ALB, TAT, TDO2, TTR, G6PC, NTCP, CYP1A2, CYP2B6, CYP2C9, CYP2C19, CYP2D6, CYP3A4, and CYP7A1 (Figs. 15 to 27).

### (Example 14)

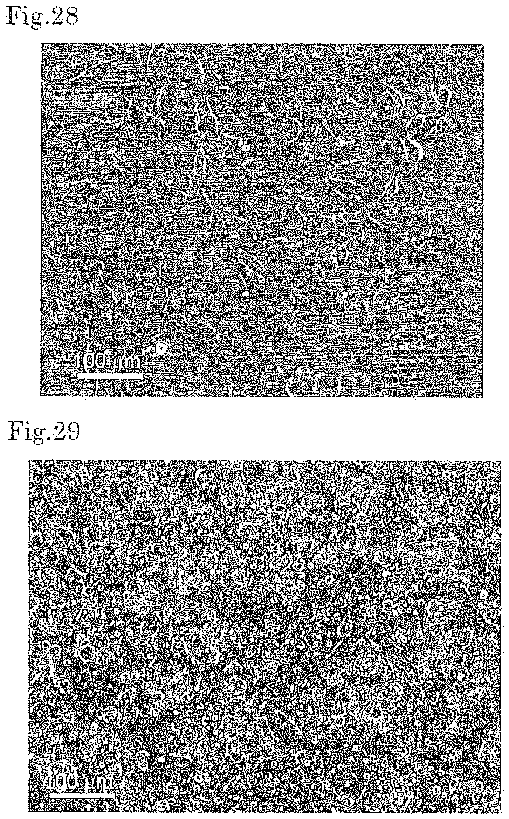

In a similar way as in Example 1, frozen human hepatocytes (10 M, Female, Hispanic; manufactured by Celsis) were used to prepare liver progenitor cells. After that, cDNA-uPA/SCID mice (2 to 4 M, Male; manufactured by PhoenixBio Co., Inc.) were subjected to laparotomy under isoflurane anesthesia, the spleen was exposed, 0.5 × 10⁵ to 2 × 10⁶ liver progenitor cells per mouse were implanted, and then the abdomen was sutured. On day 73 after implantation, the liver was removed and perfused. Hepatocytes were separated, and cultured for 4 days on 24-well collagen plates (manufactured by IWAKI) at 4 × 10⁵ cells/well using 2% FBS-SHM medium. Figs. 28 and 29 represent a photograph of the liver progenitor cells before transplantation and a photograph of the cells that were taken out from the mouse having the liver progenitor cells transplanted thereinto and cultured for 4 days, respectively. Morphological observations suggested that the implanted liver progenitor cells completely matured into hepatocytes in the mouse liver.

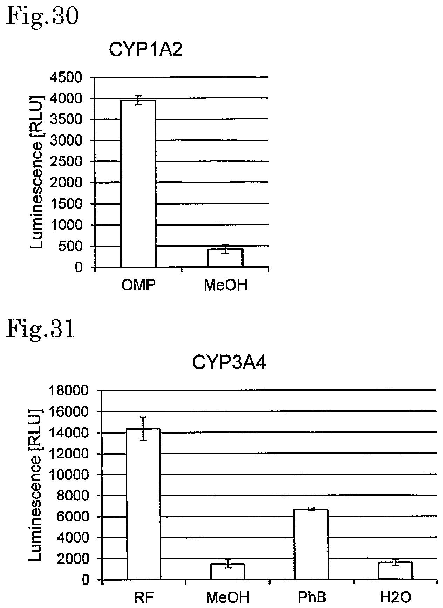

For the hepatocytes taken out from the mice, the activity of the metabolic enzyme CYP1A2 was measured using methanol (MeOH, at a concentration of 1%) and omeprazole (OMP, 50 µM). In addition, the activity of the metabolic enzyme CYP3A4 was measured using rifampicin (RF, 10 µM), methanol (MeOH, at a concentration of 1%), phenobarbital (1 mM), and distilled water. The activities of these metabolic enzymes were measured with Luciferin 1A2 kit and Luciferin-IPA kit from Promega, respectively. It was revealed that also for the liver progenitor cells which had been transplanted into the mouse liver and were cultured, CYP1A2 was induced with omeprazole, and CYP3A4 with rifampicin and phenobarbital (Figs. 30 and 31).

### (Example 15)

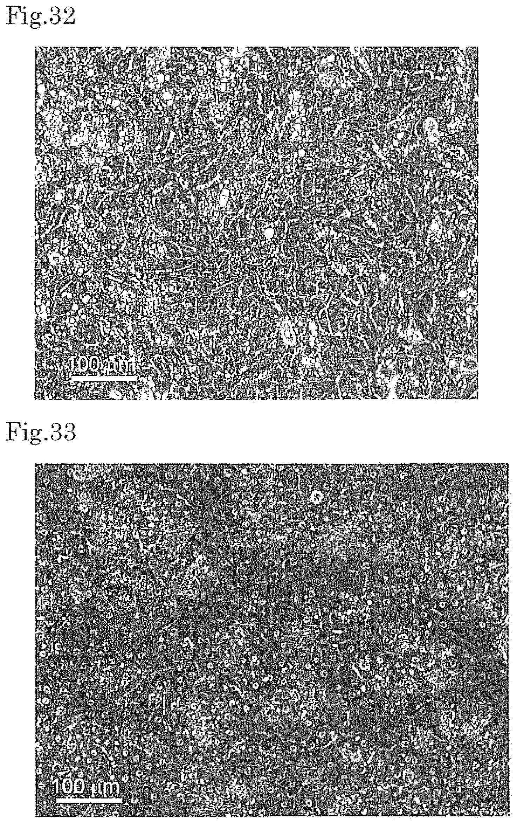

In a similar way as in Example 1, frozen human hepatocytes (8 M, Male, Caucasian; manufactured by Bioreclamation IVT) were used to prepare liver progenitor cells. The liver progenitor cells were subjected to culturing and transplantation as in Example 14. Figs. 32 and 33 represent a photograph of the liver progenitor cells before transplantation and a photograph of the cells that were taken out from the mouse having the liver progenitor cells transplanted thereinto and cultured for 4 days, respectively.

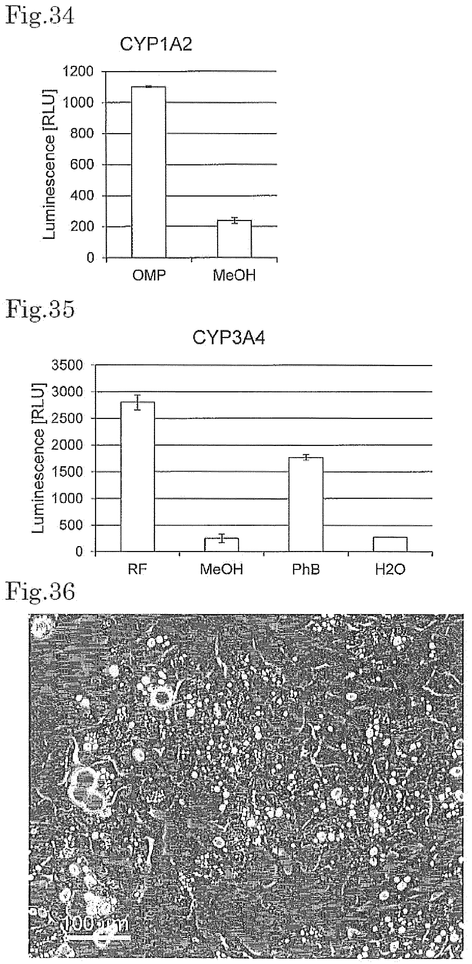

The cells taken out from the mice were subjected to measurement of the activity of metabolic enzymes as in Example 14. It was revealed that as in Example 14, CYP1A2 was induced with omeprazole, and CYP3A4 with rifampicin and phenobarbital (Figs. 34 and 35).

### (Example 16)

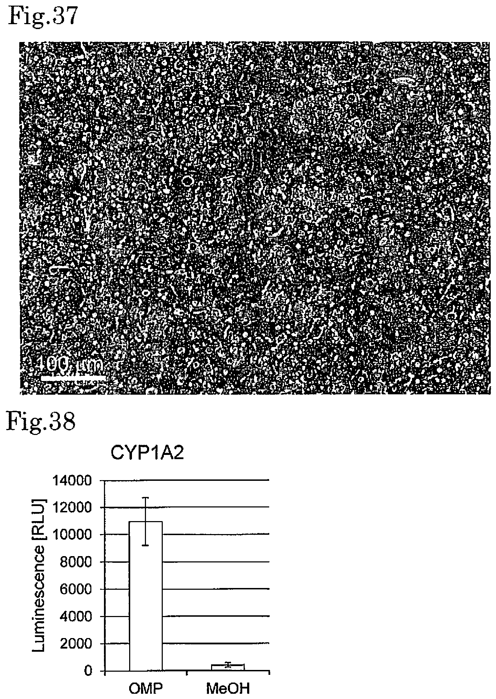

In a similar way as in Example 1, frozen human hepatocytes (1 Y, Male) were used to prepare liver progenitor cells. The liver progenitor cells were subjected to culturing and transplantation as in Example 14. Figs. 36 and 37 represent a photograph of the liver progenitor cells before transplantation and a photograph of the cells that were taken out from the mouse having the liver progenitor cells transplanted thereinto and cultured for 4 days, respectively.

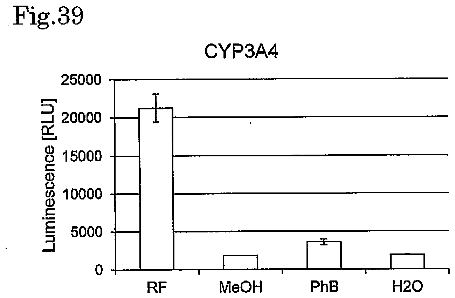

The cells taken out from the mice were subjected to measurement of the activity of metabolic enzymes as in Example 14. It was revealed that as in Example 14, CYP1A2 was induced with omeprazole, and CYP3A4 with rifampicin and phenobarbital (Figs. 38 and 39).

## Claims

1. A method for preparing human liver progenitor cells, comprising culturing human mature hepatocytes in a medium containing serum, A-83-01, and CHIR99021.

2. The method for preparing human liver progenitor cells according to claim 1, wherein the mature hepatocytes are derived from an infant or a toddler.

3. The method for preparing human liver progenitor cells according to claim 1 or 2, wherein the serum is fetal bovine serum.

4. A human liver progenitor cell prepared by culturing human mature hepatocytes in a medium containing serum, A-83-01, and CHIR99021.

5. A mature hepatocyte derived from the human liver progenitor cell according to claim 4.

6. A method for screening test substances, comprising using the mature hepatocyte according to claim 5.

7. A method for culturing a hepatitis virus, comprising using the mature hepatocyte according to claim 5.

8. A model animal of human liver in which the mature hepatocyte according to claim 5 has been transplanted into a non-human mammal.

9. A kit for evaluating metabolism and/or hepatotoxicity of a test substance using human liver progenitor cells or mature hepatocytes, the kit comprising human liver progenitor cells prepared by culturing human mature hepatocytes in a medium containing serum, A-83-01, and CHIR99021, or mature hepatocytes derived from the human liver progenitor cells.
